(19) Europäisches Patentamt
European Patent Office
Office européen des brevets

(11) Publication number: **0 009 075**
**A1**

# (12) EUROPEAN PATENT APPLICATION

(21) Application number: 79102136.3

(22) Date of filing: 27.06.79

(51) Int. Cl.[3]: **C 07 D 213/85**, C 07 D 405/12, A 61 K 31/44 // C07C91/18

(30) Priority: 27.06.78 US 919589

(43) Date of publication of application: 02.04.80 Bulletin 80/7

(84) Designated Contracting States: AT BE CH DE FR GB IT LU NL SE

(71) Applicant: **Merck & Co., Inc., Patent Department 126 East Lincoln Avenue, Rahway, N.J. 07065 (US)**

(72) Inventor: **McClure, David Earl, 2307 Bramblegate Drive, Hatfield, Pennsylvania 19440 (US)**
Inventor: **Baldwin, John James, 1680 Wagon Wheel Lane, Lansdale, Pennsylvania 19446 (US)**

(74) Representative: **Abitz, Walter, Dr.-Ing. et al, Abitz, Morf, Gritschneder P.O.Box 860109, D-8000 München 86 (DE)**

(54) **Pyridyloxy-propanol amines and esters thereof, process for preparing the same and pharmaceutical compositions containing them.**

(57) Compounds having the formula

(A) 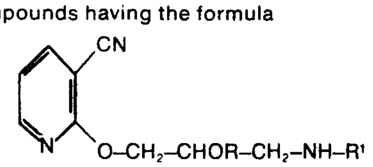 or

(B) 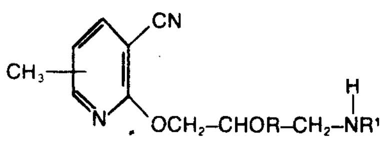

and pharmaceutically acceptable salts thereof wherein

R is H or $-\overset{O}{\overset{\|}{C}}-L$ wherein

L is $C_1$–$C_{10}$ alkyl, phenyl or mono- or di-substituted phenyl wherein said substituents are $C_1$–$C_4$ alkyl, $C_1$–$C_4$ alkoxy or halo and

R' is a phenyl $C_3$–$C_6$ branched alkyl group having the formula

(a) 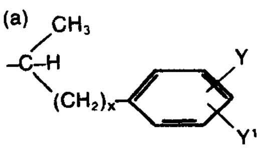

(c) 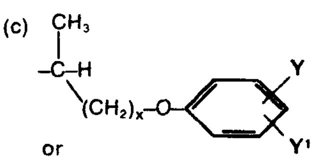

or

(b) 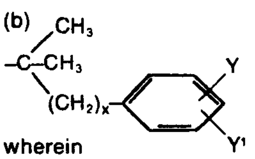

(d) 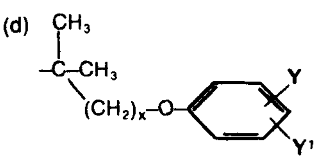

wherein

x is 1 to 3 and

Y and Y' when separate, are H, $-OCH_3$, OH or halo and, when joined, form

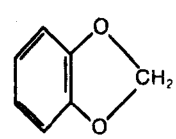 or 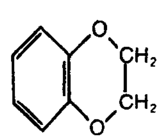

with the phenyl group, their preparation and pharmaceutical activity.



ACTORUM AG

TITLE MODIFIED
see front page



# N-ARALKYLAMINO-OR-PROPOXYCYANOPYRIDINES, PROCESS FOR PREPARING THE SAME AND PHARMACEUTICAL COMPOSITIONS CONTAINING THE SAME

## SUMMARY OF THE INVENTION:

N-phenylbranchedalkyl-OR-propoxycyano-pyridines, their preparation and use as cardiovascular active pharmaceutical agents.

## BACKGROUND OF THE INVENTION:

The present invention is concerned with N-aralkylamino-OR-propoxy cyanopyridines their preparation and their use as pharmaceutical.

N-alkylamino-OR-propoxy cyanopyridines are disclosed in U.S. 4,000,282 and U.S. 4,053,605. These compounds have pharmaceutical activity e.g., as anti-hypertensives or β-adrenergic blocking agents. Other N-substituted amino hydroxy propoxy substituted heterocyclics are disclosed in South African patent 741070 and Belgian patent 858,367; these heterocyclic compounds are also taught to have pharmaceutical activity.

Certain N-arbranchedalkyl-OR-propoxy cyanopyridines have been discovered. These compounds have pharmaceutical activity.

DESCRIPTION OF THE PREFERRED EMBODIMENTS

An embodiment of the present invention is compounds having the formula

$R_a-O-CH_2-CHOR-CH_2-NHR^1$

(I)

and pharmaceutically acceptable salts thereof wherein

$R_a$ is 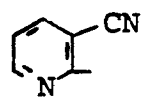 or 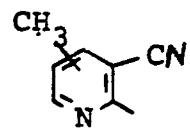

R is H or $-\overset{\overset{O}{\|}}{C}-L$ wherein

L is $C_1$-$C_{10}$ alkyl, phenyl or mono-or di-substituted phenyl wherein said substituents are $C_1$-$C_4$ alkyl, $C_1$-$C_4$ alkoxy or halo and

$R^1$ is a phenyl $C_3$-$C_6$ branched alkyl group having the formula

(a) 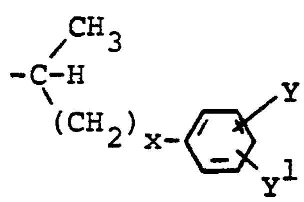

(b) 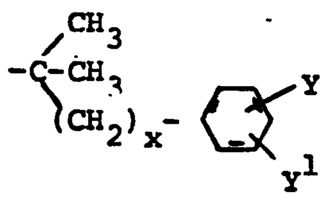

(c) 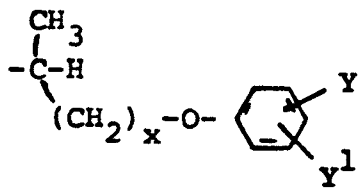

or

(d) 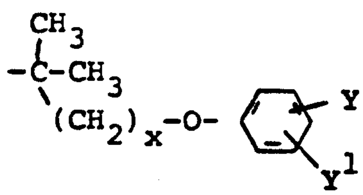

wherein

x is 1 to 3 and

Y and $Y^1$, when separate, are H, $-OCH_3$, OH or halo and when joined, form

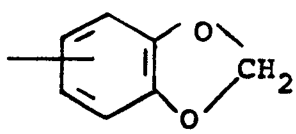 or 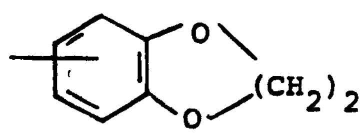

with the phenyl group.

$R_1$ is H or the $-\overset{O}{\overset{\|}{C}}-L$ group, with H being preferred. The L group includes $C_1$-$C_{10}$, linear and branched hydrocarbon alkyl such as methyl, n-decyl, <u>tert</u>-butyl, isoamyl, n-heptyl and the like with $C_1$-$C_4$ alkyl being preferred, and phenyl or mono- and disubstituted phenyl such as <u>tert</u>-butylphenyl, 2,6-

dibromophenyl, 3-methylphenyl, 4-n-propylphenyl, 3,5-dimethoxyphenyl, 4-iodophenyl, 2-methyl-4-chlorophenyl, 4-fluorophenyl and the like, with monosubstituted phenyl preferred.

The alkylidene group, $-(CH_2)_x-$may have from 1 to 3 units, with 1 to 2 being preferred.

Y and $Y^1$ may be H, $-OCH_3$, -OH or halo. The halo substituent includes Cl, Br, I or F with Cl and F being preferred.

Y and $Y^1$ may also be joined to form the dioxy alkylene moiety $-O-(CH_2)_{1\ or\ 2}-O-$ which is attached to the phenyl ring to form a bicyclic group of the formula

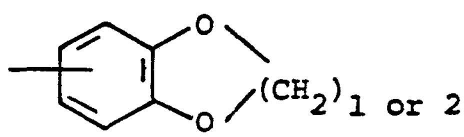

Preferred positioning of the dioxyalkylene moiety is

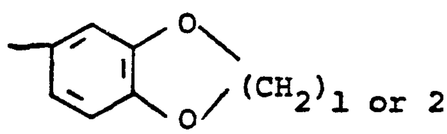

One class of preferred compounds has the formula

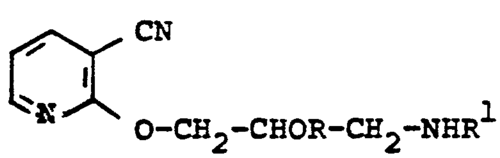

Ia

Another class of preferred compounds has the formula

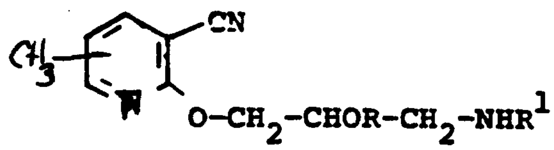

Ib

especially where $-CH_3$ is in the 5-position in the pyridine ring.

Other classes of preferred compounds are those of formula I, Ia, or Ib where

$R_1$ is

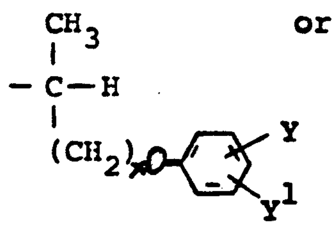

or

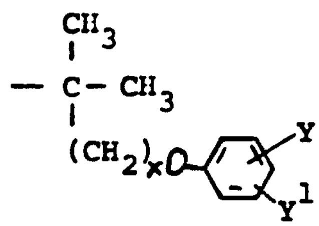

In more preferred compounds of this class, x is 1.

Another class of preferred compounds are those of formula I Ia or Ib where

$R_1$ is

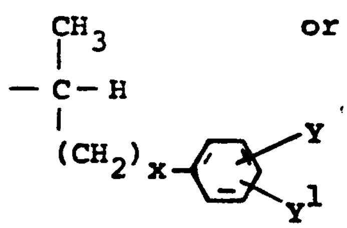

or

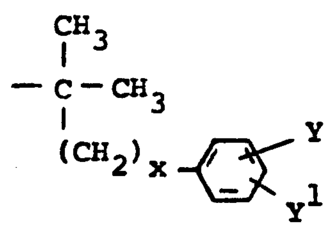

In more preferred compounds of this class, x is 2 and Y and $Y^1$ are selected from H and $-OCH_3$, and especially where Y is H and $Y^1$ is $OCH_3$, Y and $Y^1$ are both $OCH_3$, or Y and $Y^1$ are both H, or Y and $Y^1$ are joined to form $-O-(CH_2)_{1\ or\ 2}-O-$ and more preferably positioned

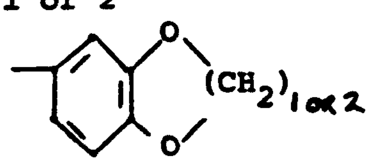

in the phenyl ring.

All the optical isomer forms, that is mixtures of enantiomers or diastereomers, e.g., racemates as well as the individual enantiomers or diastereomers of Formula I are included. These individual enantiomers of Formula I are commonly designated according to the optical rotation they effect by (+) and (-), (L) and (D), (l) and (d) or combinations of these symbols. These isomers may also be designated according to their absolute spatial configuration by (S) and (R) which stand for sinister and rectus, respectively. Where no symbol is used in naming a compound, the compound is a racemate. The (S) isomer is a preferred isomer configuration.

The pyridines of formula I, Ia or Ib have one chiral center at the 2-position in the propoxy substituent and can have a second chiral center when the alkylene group in $R^1$ is

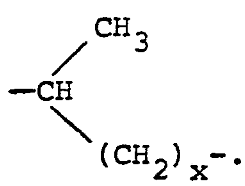

The aforesaid symbols, e.g., S, R or (R, S), when used to designate a Formula I compound, refer only to the optical configuration around the chiral center at the 2-position in the propoxy substituent.

The pyridines of the present invention can be prepared by any convenient process.

One such process involves the coupling of a halopyridine with a (i) suitable substituted oxazolidine and hydrolyzing the reaction product obtained or (ii) suitable gylcolamine. These processes are illustrated by the following reaction equations:

(i)

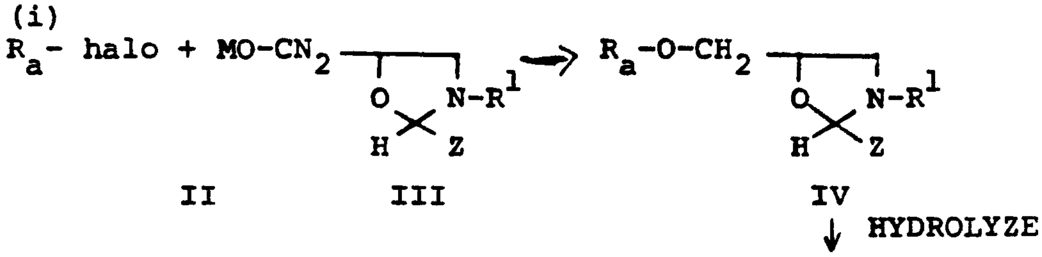


II III IV

(ii)

$$Ra\text{- halo} + HO\text{-}CH_2\text{-}CHOH\text{-}CH_2\overset{H}{N}R^1 \xrightarrow{base} R_a\text{-}O\text{-}CH_2\text{-}CHOH\text{-}CH_2\text{-}NHR^1$$

II (a glycolamine)

Halo may be Cl, Br, F or I, with Cl being preferred. M is an alkali metal, either potassium or sodium. Z can be hydrogen or the residue of any suitable aldehyde

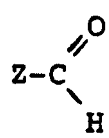

e.g., an arylaldehyde, such as benzaldehyde, naphthaldehyde and the like, or an alkanal such as acetaldehyde, butyraldehyde and the like. The process for preparing oxazolidines where M is hydrogen is disclosed in U.S.P. 3,718,647 and U.S.P. 3,657,237 and to the extent necessary the pertinent disclosure is incorporated herein by reference. The alkali metal salt of the oxazolidine is prepared in a conventional manner by reaction of the corresponding hydroxymethyloxazolidine with an appropriate amount

of an alkali base reactant. However, this reaction (i) may also be carried out with in situ formation of the alkali metal oxazolidine salt (Formula III) by reacting the oxazolidine

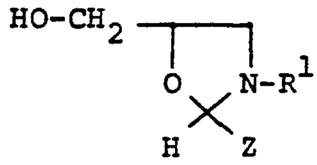

V

with the Formula II pyridine in the presence of a strong base as used in reaction (ii) such as an alkali metal alkoxide (e.g., $K-O-C-(CH_3)_3$) or sodium hydride.

The coupling reactions can be carried out at temperatures ranging from about 0°C. to the reflux temperature of the solvent. A temperature range of about 10°C. to about 75°C. is preferred. The reaction is generally carried out in a solvent. Any suitable solvent may be used. Examples of useful solvents are dimethylformamide, dimethylsulfoxide, hexamethyl-phosphoramide, tert butanol, alkanols and the like. The hydrolysis is carried out using conventional acid hydrolysis reagents and techniques, e.g., treatment with a solution of an acid such as acetic acid or any strong mineral acid such as HCl or $H_2SO_4$. The hydrolysis product can be directly obtained as the salt of the acid used for the hydrolysis. Ordinarily, the product I is recovered as the free base after conven-tional neutralization of the salt.

The coupling reaction is ordinarily carried out at atmospheric pressure. Higher pressures may be used if desired.

When a racemic oxazolidine (Formula III or V is used as a reactant, the product is obtained as a racemate. The racemate may be separated into its individual enantiomers by conventional resolution techniques, e.g., using an enantiomer of a suitable optically active organic acid such as tartaric acid.

When Z in the oxazolidine, i.e., Formula III, IV or V, is other than hydrogen, in addition to the chiral center at oxazolidine position 5 there is a second chiral center at position 2 and a third chiral center may arise when $R^1$ contains the $-C(CH_3)(H)-(CH_2)_x-$ group . However, whenever the oxazolidine is designated, e.g., as (S), (R) or (R,S), this designation refers only to the optical configuration around the carbon atom at the 5-position.

By using a single optical isomer of said oxazolidine or said glycolamine in the above reactions, the product may be obtained directly as a single enantiomer. Thus, e.g., if the S-isomer of the oxazolidine is used, then the product obtained will be the S-isomer. This provides a convenient way for directly preparing individual isomers of the present pyridines.

Another convenient process for preparing the present pyridines is by treating an appropriate pyridine epoxide with a suitable amine as illustrated by the following reaction equation:

$$R_a-O-CH_2-\overset{O}{\overbrace{CH-CH_2}} + R^1NH_2 \rightarrow R_a-O-CH_2-CHOH-CH_2NHR^1$$

VI I'

This reaction is advantageously carried out in excess amine ($R^1NH_2$) reactant. Temperatures up to reflux can be used. An especially preferred reaction temperature range is room temperature to about 100°C. The reaction is conveniently carried out at room temperature.

The product from the epoxide/$R^1NH_2$ reaction is ordinarily a racemate, and can be separated using conventional resolution procedures.

If a single optical isomer of the formula VI epoxide is used, as the reactant, the product obtained is the corresponding single optical iosmer, e.g., (S)-VI + $R^1NH_2$ ⟶ (S)-I.

The optically active epoxide intermediates of formula VI can be prepared by the reaction illustrated below

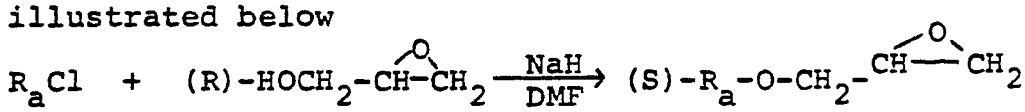


VII

; conventional reaction conditions are used. If the (S) isomer of formula VII is used, the corresponding (R) isomer of formula VI is obtained. Preparation of the intermediates will be shown in the examples below.

Pyridines of the present invention wherein R is other than hydrogen are conveniently prepared by treating the corresponding pyridine where R is hydrogen with an appropriate acylating agent such as an acyl halide, e.g., undecanoyl chloride, pivaloyl

chloride, benzoylchloride, p-methoxybenzoyl chloride or an anhdyride, e.g., acetic anhydride, and the like. The reaction is illustrated by the following equation:

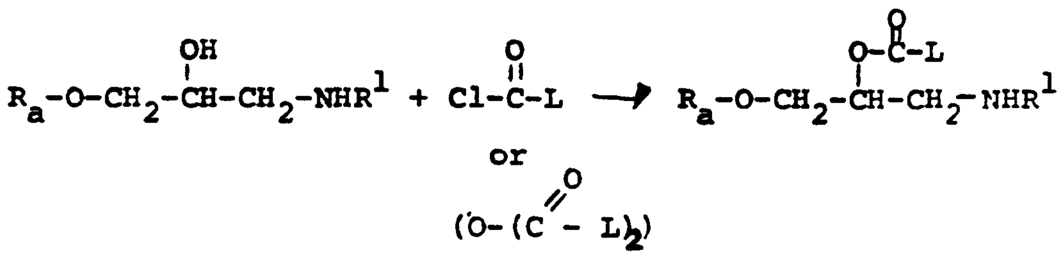


The compounds of the present invention also include the pharmaceutically acceptable salts of the present pyridines. These salts are generally salts of the Formula I pyridines and organic or inorganic acids. These salts are prepared by treating the pyridine with an appropriate amount of a useful acid, generally in a suitable solvent. Examples of useful organic acids are isothionic acid and carboxylic acids such as maleic acid, acetic acid, tartaric acid, propionic acid, fumaric acid, succinic acid, pamoic acid, oxalic acid, pivalic acid and the like; useful inorganic acids are hydrohalo acids such as HCl, HBr, HI; sulfuric acid, phosphoric acid and the like. The hydrochloride and hydrogen maleate salts are examples of preferred salts.

The compounds of the present invention have β-adrenergic blocking activity. This β-adrenergic blocking activity is determined by measuring the ability of representative pyridines to block the β-adrenergic stimulant effect of isoproterenol in a test animal.

The compounds of the present invention also have α-adrenergic blocking activity. This α-adrenergic blocking activity is determined, (a) in vitro by mesuring the ability of a representative pyridine to displace radio labeled α-adrenergic antagonist from a tissue substrate or (b) in vivo, by measuring the ability of representative pyridines to block the α-adrenergic stimulant effect of phenylephrine in anesthetized normotensive animals.

The present pyridines also exhibit antihypertensive activity of immediate onset. This rapid onset antihypertensive activity is determined by administering a representative pyridine of the present invention to spontaneously hypertensive (SH) rats and measuring the effect on blood pressure.

The α- and β-adrenergic blocking activity of the present pyridines indicates that the compounds may be useful for treating cardiovascular conditions susceptible to β-blockade therapy (e.g., angina pectoris, arrhythmia) while minimizing bronchocon-striction via α-adrenergic blockade. This dual α/β-blockade effect may also be useful in treating hypertension caused by pheochromocytoma.

For use as α/β-adrenergic blocking agents, and/or antihypertensive agents the compounds of the present invention can be administered orally, by inhalation, by suppository or parenterally, i.e., intravenously, intraperitoneally, etc. and in any suitable dosage form. The compounds may be offered in a form (1) for oral administration, e.g., as tablets in combination with other compounding

ingredients (diluents or carriers) customarily used such as talc, vegetable oils, polyols, benzyl alcohols, starches, gelatin and the like - or dissolved, dispersed or emulsified in a suitable liquid carrier - or in capsules or encapsulated in a suitable encapsulating material, or (2) for parenteral administration, dissolved, dispersed, or emulsified in a suitable liquid carrier or diluent or (3) as an aerosol or (4) as a suppository. The ratio of active ingredient (present pyridine) to compounding ingredients will vary as the dosage form requires. Conventional procedures are used to prepare the pharmaceutical formulations.

The effective daily dosage level for the present compounds may be varied from about 100 mg. to about 3000 mg. Daily doses ranging from about 200 to about 2500 mg. are preferred, with about 300 to about 1000 mg. being a more preferred range. Oral administration is preferred. Either single or multiple daily doses may be administered depending on unit dosage.

Thus, another embodiment of this invention is a pharmaceutical composition containing a therapeutically effective amount of a compound of the present invention.

The following examples illustrate the preparation of representative compounds of the present invention. Temperatures are in ° Celsius.

## EXAMPLE 1

3-(1,1-dimethyl-3-phenylpropylamino)-1,2-dihydroxypropane

To 1,1-dimethyl-3-phenylpropylamine (3.6 g., 22 mmole) in 20 ml. 2-propanol at 50°C. in an oil bath was added glycidol (1.7 g., 23 mmole) in 10 ml. 2-propanol dropwise over 5-10 min. The mixture was heated at 75°C. for 2 hours. After evaporation of the solvent, vacuum distillation provided 3.9 g. (75%) of 3-(1,1-dimethyl-3-phenylpropylamino-1,2-dihydroxypropane (bp 160-180° at 0.5 torr), which crystallized upon standing (mp 74-77°).

## EXAMPLE 2

(S)-2-[3-(1,1-dimethyl-3-phenylpropylamino)-2-hydroxy-1-propoxy]-3-cyanopyridine maleate

To an ice-cooled solution of 41.0 g. (0.25 mole) of 1,1-dimethyl-3-phenylpropylamine in 70 ml. methanol with 5.0 g. of 5% Pd/C was added an ice-cooled solution of 11.7 g. (0.09 mole) of isopropylidene-(R)-glyceraldehyde in 130 ml. THF over 1 hour under a 40 psi atmosphere of hydrogen. After uptake of $H_2$ had ceased, the mixture was filtered, washed with methanol, and concentrated. The residue was heated in 50 ml. 6N HCl and 50 ml. acetone for 1/2 hour on a steam bath. The mixture was cooled and made basic with NaOH. Extraction with methylene chloride and ethyl acetate, followed by drying ($Na_2SO_4$) and concentration yielded a residue which on trituration with petroleum ether followed by filtration gave 12.8 g. (60%) of (S)3-(1,1-dimethyl-3-phenylpropylamino)-1,2-dihydroxypropane.

A solution of 4.5 g. (19 mmole) of the glycolamine from above, 30 ml. benzaldehyde, 18 ml. benzene, and 200 mg. benzoic acid was refluxed for 3 hours while collecting the water in a Dean-Stark trap. The solution was diluted with benzene and washed with aqueous potassium carbonate. The mixture was concentrated and then heated at 75-80°C./ 0.2 torr to remove any volatile materials.

The residue from above in 50 ml. DMF was added dropwise to sodium hydride (1.0 g. of a 50% oil dispersion washed with hexane) in 25 ml. DMF. Stirring was continued for 1 hour, and then 2.60 g. (19 mmole) of 2-chloro-3-cyanopyridine in 25 ml. DMF was added. After stirring overnight at room temperature, most of the DMF was evaporated. Water was added and washed with ether. The ether was washed with a mixture of 1N HCL and methanol (2:1). The acid washings were heated on the steam bath for 1/2 hour. After washing with ether, the mixture was basified with $K_2CO_3$. The oil was extracted with $CH_2Cl_2$, dried ($Na_2SO_4$), and concentrated to give 6.4 g. (97%) crude free base. The maleate salt was prepared in 2-propanol, and recrystallization from 2-propanol/methanol gave pure (S)-2-[3-(1,1-dimethyl-3-phenylpropylamino)-2-hydroxy-1-propoxy]-3-cyanopyridine maleate, mp 142-145°C.

## EXAMPLE 3

(S)-2-[3-(1,1-dimethyl-2-(4-methoxyphenyl)ethylamino)-2-hydroxy-1-propoxy]-3-cyanopyridine maleate

To an ice-cooled solution of 29 g. (0.16 mole) of 1,1-dimethyl-2-(4-methoxyphenyl)ethylamine in 75 ml. of methanol with 4.0 g. of 5% Pd/C was

added an ice-cooled soltuion of 8.0 g. (0.062 mole) of isopropylidene-(R)-glyceraldehyde in 85 ml. of THF over 1 hour under a 40 psi atmosphere of hydrogen. After uptake of $H_2$ had ceased, the mixture was filtered, washed with methanol, and concentrated. The residue was heated in 50 ml. of 6N HCl and 50 ml. acetone for 1/2 hour on a steam bath. The mixture was made basic with NaOH, and extracted with ethyl acetate. After drying and concentration, trituration of the residue with petroleum ether led to crystallization. Filtration gave 8.3 g. (56%) of (S)-3-[1,1-dimethyl-2-(4-methoxyphenyl)ethylamino)-1,2-dihydroxypropane which was dissolved in 50 ml. DMF for use in the next step.

The DMF solution from above was added dropwise to 1.6 g. of sodium hydride (50% oil dispersion washed with hexane) in 50 ml. DMF. Stirring was continued for 45 minutes, and then 4.4 g. (0.032 mole) of 2-chloro-3-cyanopyridine in 50 ml. DMF was added. After stirring overnight at room temperature, most of the DMF was evaporated. Water was added and washed with $CH_2Cl_2$ which was dried ($Na_2SO_4$) and concentrated to give 6.4 g. (56%) of the crude free base of the title compound.

A gradient elution chromatography of this material on silica gel eluting with methanol-chloroform mixtures gave the desired free base which eluted with 3% methanol/chloroform. The maleate salt was prepared in 2-propanol/ether, and recrystallization from 2-propanol gave pure (S)-2-[3-(1,1-dimethyl-2-(4-methoxyphenyl)ethylamino)-2-hydroxy-1-propoxy]-3-cyanopyridine maleate, mp 126-130°C.

EXAMPLE 4

2-(2,3-epoxy-1-propoxy)-3-cyanopyridine

A solution of 22.4 g. (0.17 mole) of 2,2-dimethyl-1,3-dioxolane-4-methanol in 100 ml. DMF was added to 8.2 g. of sodium hydride (50% oil dispersion) in 50 ml. DMF dropwise over 1 hour, and stirring was continued for an additional hour. A solution of 19.4 g. (0.14 mole) of 2-chloro-3-cyanopyridine in 100 ml. DMF was added dropwise over 1/2 hour, and the mixture was stirred at room temperature overnight. Water was added and then extracted with $CH_2Cl_2$. After drying ($Na_2SO_4$) and concentration, petroleum ether was added and crystallization occurred. Filtration gave 2-(2,2-dimethyl-1,3-dioxolane-4-methoxy)-3-cyanopyridine (mp 61-64°C.), which was cleaved to the corresponding diol by heating in 100 ml. of 3<u>N</u> HCl/100 ml. acetone for 1/2 hour on a steam bath.

To an ice-cooled solution of 21.4 g. (0.11 mole) of the diol from above in 50 ml. pyridine was added 12.6 g. (0.11 mole) of methanesulfonyl chloride dropwise over 30 minutes. After stirring for an additional 30 minutes without cooling, the mixture was diluted with 250 ml. of $CH_2Cl_2$ and then cooled in an ice bath. A solution of 11.3 g. (0.21 mole) of sodium methoxide in 50 ml. of methanol was added over a few minutes. Cooling was discontinued, and the mixture was stirred for 1 hour. Washing with water, cold dilute HCl, and saturated $NaHCO_3$, followed by drying ($Na_2SO_4$) and concentration

gave 16.0 g. (70%) of crude 2-(2,3-epoxy-1-propoxy)-3-cyanopyridine, which crystallized upon standing. This could be used in subsequent steps without further purification or purified according to the procedure in Example 5.

## EXAMPLE 5

2-(2,3-epoxy-1-propoxy)-3-cyanopyridine

To an ice-cooled mixture of 2.2 g.(16 mmole) of 2-chloro-3-cyanopyridine and 0.85 g. of sodium hydride (50% oil dispersion) in 25 ml. of DMF was added 1.20 g. (16 mmole) of glycidol in 10 ml. of DMF dropwise over 1 hour, and then stirring was continued overnight. An equal amount of water was added and extracted with ether. The ether layer was dried ($Na_2SO_4$) and concentrated. This crude product was triturated with hot hexane and decanted, repeating the process several times. The combined hexane washes were cooled to -30°C. and then filtered to give 1.4 g. (50%) of 2-(2,3-epoxy-1-propoxy)-3-cyanopyridine, mp 55-57°C.

EXAMPLE 6a

(S)-2-(2,3-epoxy-1-propoxy)-3-cyanopyridine

To an ice-cooled mixture of 8.0 g. (56 mmole) of 2-chloro-3-cyanopyridine and 3.1 g. sodium hydride (50% oil dispersion) in 20 ml. of DMF was added an ice-cooled solution of 4.2 g. (57 mmole) of (R)-glycidol in 40 ml. of DMF over 1 hour, and then the mixture was stirred overnight while warming to room temperature. An equal amount of water was added and extracted with ether, which was dried ($Na_2SO_4$) and concentrated. The crude product was triturated with hot hexane and decanted, repeating the process several times. The combined hexane washes were cooled to 5°C. and filtered to give 5.5 g. (55%) of (S)-2-(2,3-epoxy-1-propoxy)-3-cyanopyridine, mp 68-70°C.

EXAMPLE 6b

(S)-2-(2,3-epoxy-1-propoxy)-3-cyanopyridine

To an ice-cooled mixture of 2-chloro-3-cyanopyridine (1.39 g, 10 mmole) and NaH (1.0 g, 50% oil dispersion, 21 mmole) in 25 ml DMF was added (R)-3-tosyloxy-1,2-propanediol (2.5 g, 10 mmole) in 50 ml DMF dropwise over 1.5 hr. The mixture was allowed to stir overnight while slowly warming to room temperature. Workup as presented in Example 6a gave (S)-2-(2,3-epoxy-1-propoxy)-3-cyanopyridine, (0.68 g, 39%); mp 64-66°C.

(R)-2-(2,3-epoxy-1-propyoxy)-3-cyanopyridine can be prepared from 2-chloro-3-cyanopyridine and either (S)-3-tosyloxy-1,2-propanediol or (S)-glycidol according to the procedures described for the corresponding (S)-isomer in Examples 6a or 6b.

## EXAMPLE 7

2-[3-(1,1-dimethyl-3-phenylpropylamino)-2-hydroxy-1-propoxy]-3-cyanopyridine maleate

A mixture of 0.95 g. (5.4 mmole) of 2-(2,3-epoxy-1-propoxy)-3-cyanopyridine and 2.4 g. (15 mmole) of 1,1-dimethyl-3-phenylpropylamine was heated at 50°C. overnight. The desired product was isolated by chromatography on silica gel eluting with 5% methanol/chloroform. The maleate salt was prepared in 2-propanol/ether, and recrystallization from 2-propanol gave pure 2-[3-(1,1-dimethyl-3-phenylpropylamino)-2-hydroxy-1-propoxy]-3-cyanopyridine maleate, mp 134-135°C.

Other compounds prepared according to the Example 7 procedure are included in the following table.

TABLE I

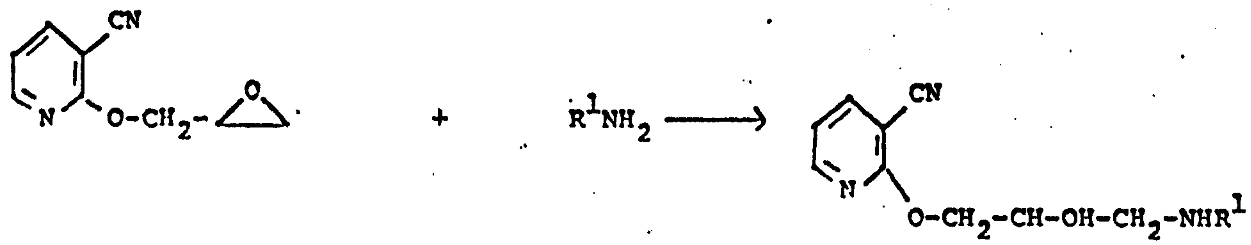

(A) (B) (C)

| Example | (A) Isomer | (B) Amine | TIME (hrs) | Reaction TEMP °C. | RECRYS. SOLVENT ⊕ | PRODUCT (C) ⊕⊕ ISOLATED AS | M.P. (°C) |
|---|---|---|---|---|---|---|---|
| 8 | R,S | 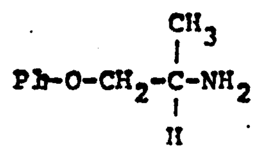 | 18 | 65 | IPA/Ether | maleate salt | 88-89° |
| 9 | R,S | 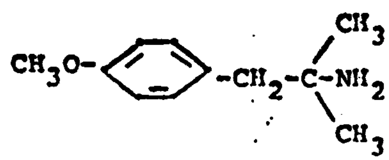 | 7 | 80 | EtOH/Ether | HCl salt | 151-153° |
| 10 | S | 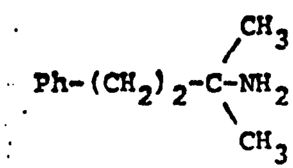 | 16 | 60 | IPA | maleate salt | 151-153 |

TABLE I, cont'd.

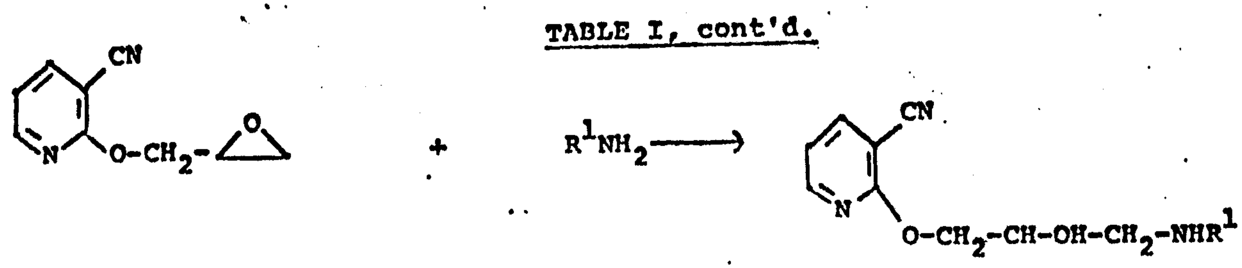

(A) (B) (C)

| Example | (A) Isomer | (B) Amine | TIME (hrs) | Reaction TEMP °C. | RECRYS.® SOLVENT | PRODUCT (C)®® ISOLATED AS | M.P. (°C) |
|---|---|---|---|---|---|---|---|
| 11 | R,S | 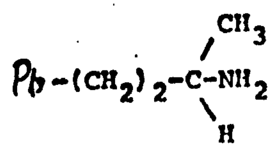 | 20 | 50 | IPA | maleate salt | 115-117° |
| 12 | S | 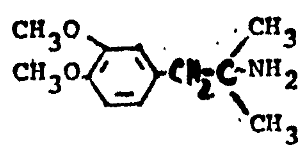 | 16 | 60 | EtOH | HCl salt | 186-189° |
| 13 | R,S | 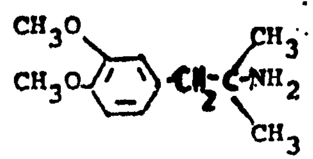 | 16 | 60 | EtOH | HCL salt | 192-195° |

TABLE I, cont'd.

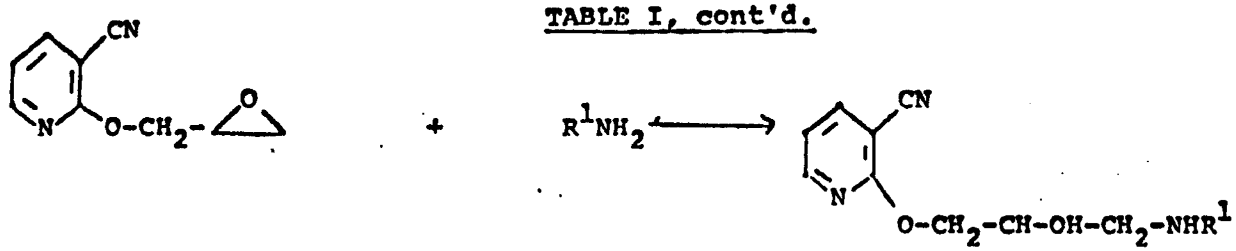

(A) (B) (C)

| Example | (A) Isomer | (B) Amine | TIME (hrs) | Reaction TEMP °C. | RECRYS.⊛ SOLVENT | PRODUCT (C)⊛⊛ ISOLATED AS | M.P. (°C) |
|---|---|---|---|---|---|---|---|
| 14 | S | 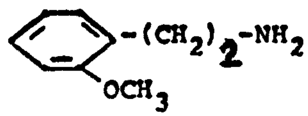 | 16 | 60 | Toluene | Free base | 97-99° |
| 15 | S | 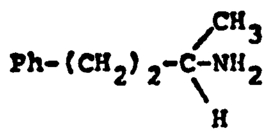 | 16 | 60 | IPA | maleate salt | 118-119° |
| 16 | R | 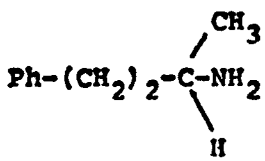 | 16 | 60 | IPA | maleate salt | 116-119° |

TABLE I, cont'd.

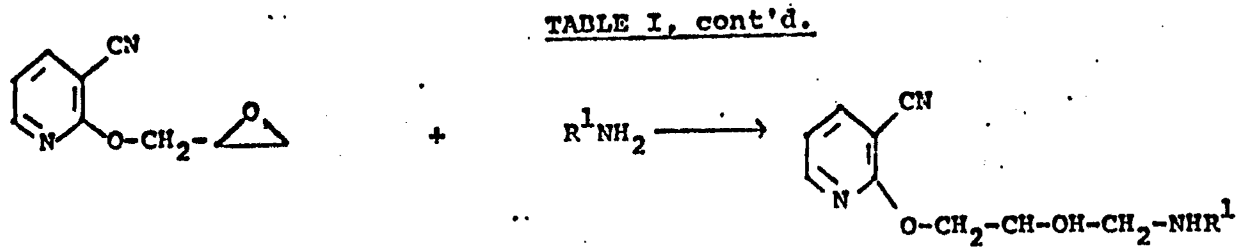

(A) (B) (C)

| Example | (A) Isomer | (B) Amine | TIME (hrs) | Reaction TEMP °C. | RECRYS.⊛ SOLVENT | PRODUCT (C)⊛⊛ ISOLATED AS | M.P. (°C) |
|---|---|---|---|---|---|---|---|
| 17 | S | 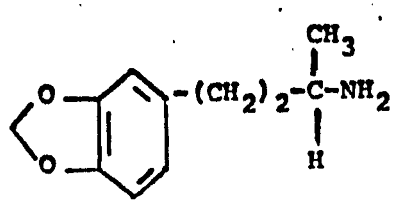 | 16 | 70 | IPA/Ether | maleate salt | 101-104° |

⊛ - IPA is isopropanol; EtOH is ethanol.

⊛⊛ - Product has same isomer designation as (A).

EXAMPLE 18

2-[3-(1,1-dimethyl)-2-(4-hydroxyphenyl)ethylamino)-2-hydroxy-1-propoxy]-3-cyanopyridine hydrochloride

A solution containing 1.41 g. (8 mmole) of 2-(2,3-epoxy-1-propoxy)-3-cyanopyridine and 1.37 g. (8 mmole) of 1,1-dimethyl-2-(4-hydroxyphenyl)ethylamine in 20 ml. acetonitrile and 5 ml. water was stirred for 1 week at room temperature. The solvent was evaporated, and the residue was taken up in 5% $CH_3OH/CH_2Cl_2$. After washing with water and drying, the organic phase was concentrated. The residue was chromatographed on silica gel eluting with 5% methanol/chloroform saturated with ammonia. The appropriate fractions were concentrated, and the hydrochloride salt prepared in 2-propanol/ether. Recrystallization from 2-propanol gave 2-[3-(1,1-dimethyl)-2-(4-hydroxyphenyl)ethylamino)-2-hydroxy-1-propoxy]-3-cyanopyridine hydrochloride; mp 193-196°C.

EXAMPLE 19

a) 2-(2,3-epoxy-1-propoxy)-3-cyano-5-methylpyridine

Followinh the procedure used in Example 8 and substituting 2-bromo-3-cyano-5-methylpyridine for 2-chloro-3-cyanopyridine, the 2-(2,3-epoxy-1-propoxy)-3-cyano-5-methylpyridine compound can be obtained in 45% yield; m.p. 74-77°C.

b) (S)-2-(2,3-epoxy-1-propoxy)-3-cyano-5-methylpyridine

Following the procedures used in Examples 6 or 6a and substituting 2-bromo-3-cyano-5-methylpyridine for 2-chloro-3-cyanopyridine, the (S)-2-(2,3-epoxy-1-propoxy)-3-cyano-5-methylpyridine compound can be obtained in 38% yield; m.p. 76-78°C.

c) 2-[3-(1,1-dimethyl-3-phenylpropylamino)-2-hydroxy-1-propoxy]-3-cyano-5-methylpyridine maleate

Following the procedure described in Example 7 and substituting 2-(2,3-epoxy-1-propyoxy)-3-cyano-5-methylpyridine for 2-(2,3-epoxy-1-propoxy)-3-cyano pyridine, the 2-[3-(1,1-dimethyl-3-phenylpropylamino)-2-hydroxy-1-propy]-3-cyano-5-methylpyridine maleate compound can be obtained; m.p. 149-151°C.

EXAMPLE 20

2-[3-(1-methyl-3-phenylpropylamino)-2-hydroxy-1-propoxy]-3-cyano-5-methylpyridine maleate

Following the procedure described in Examle 7 and substituting 2-(2,3-epoxy-1-propoxy)-3-cyano-5-methylpyridine for 2-(2,3-epoxy-1-propoxy)-3-cyanopyridine, the 2-[3-(1-methyl-3-phenylpropylamino)-2-hydroxy-1-propoxy[-3-cyano-5-methylpyridine ma;eate compound can be obtained; m.p. 90-92°C.

Claims to the invention follow.

**WHAT IS CLAIMED IS:**

1. Compounds having the formula

(A)

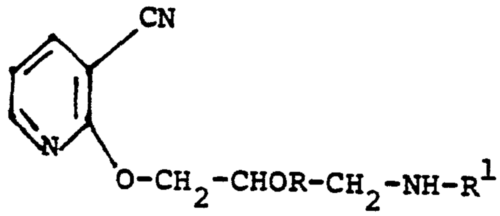

or

(B)

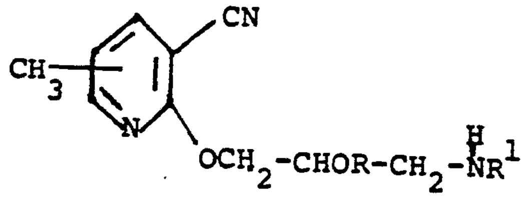

and pharmaceutically acceptable salts thereof wherein

R is H or - 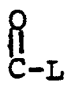 wherein

L is $C_1$-$C_{10}$ alkyl, phenyl or mono- or di-substituted phenyl wherein said substituents are $C_1$-$C_4$ alkyl, $C_1$-$C_4$ alkoxy or halo and $R^1$ is a phenyl $C_3$-$C_6$ branched alkyl group having the formula

(a)

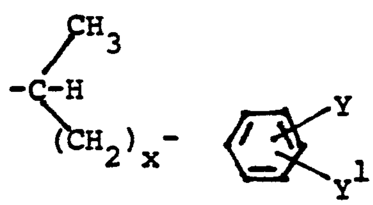

(b)

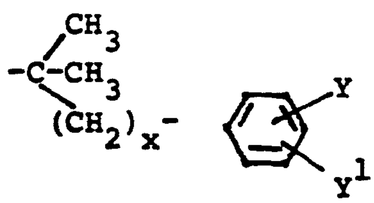

(c) 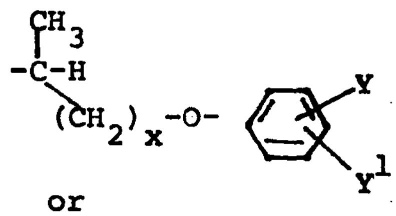

or

(d) 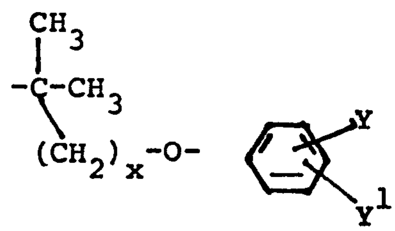

wherein

x is 1 to 3 and

Y and $Y^1$ when separate, are H, $-OCH_3$, OH or halo and, when joined, form

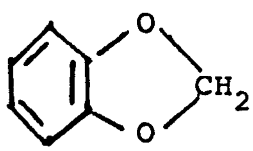 or 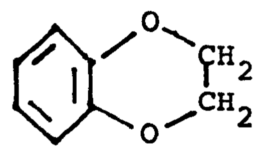

with the phenyl group.

2. Compounds of Claim 1 wherein R is $-\overset{O}{\overset{\|}{C}}-L$.

3. Compounds of Claim 1 having formula (A) wherein (i) R is H, (ii) R is H and

$R^1$ is 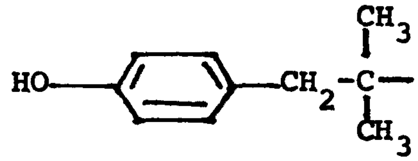 ,

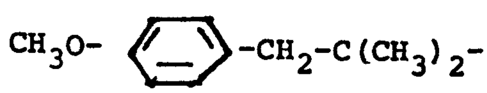 ,

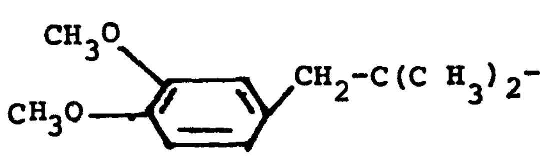

or

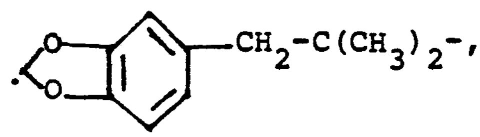, or

(iii) R is H and $R^1$ is (a) or (b).

4. Compounds of Claim 1 having formula (A) (i) wherein R is H and $R^1$ is ( a ),
(ii) R is H and

$R^1$ is 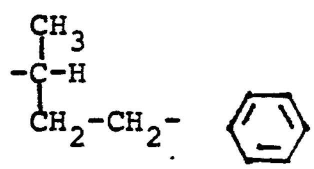, or

(iii) wherein R is H, $R^1$ is 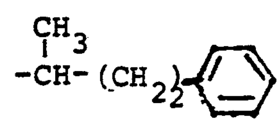 and is the S-isomer or R-isomer.

5. Compounds of Claim 1 having formula (A) wherein (i) $R^1$ is (b),

(ii) $R^1$ is 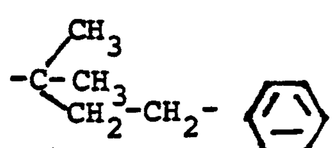, or

(iii) $R^1$ is 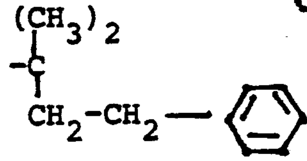 and is the S-isomer.

6. Compounds of Claim 1 (i) having formula (B) wherein (i) R is H, (ii) having formula (B) wherein R is H and $R^1$ is (a) or (b), or (iii) having formula (B) wherein R is H and

$R^1$ is 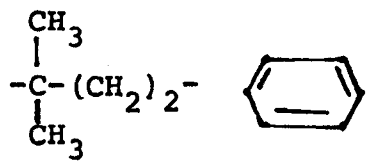 or 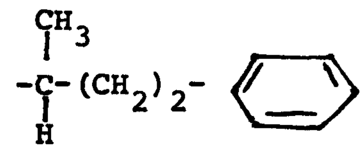

7. Compounds of Claim 6 (iii) wherein formula (B) is

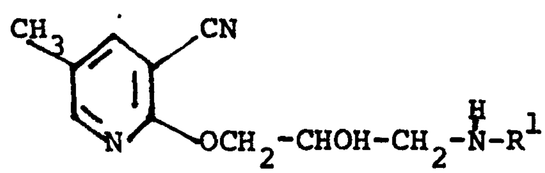

8. A pharmaceutical composition for treating hypertension containing a compound of Claim 1.

9. A process for preparing compounds of Claim 1 which comprises (a) hydrolysis of a compound of the formula

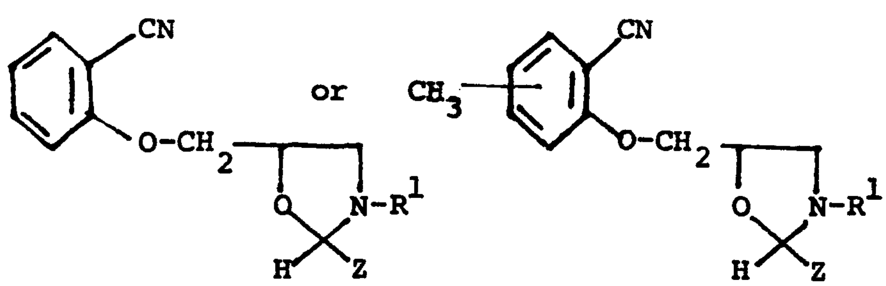

or

wherein Z is an aldehyde residue to obtain Claim 1 compounds compounds where R is H and (b) additionally treating the product from (a) with a suitable acylating agent to obtain Claim 1 compounds where R is $-\overset{O}{\overset{\|}{C}}-L$.

European Patent Office

# EUROPEAN SEARCH REPORT

0009075

Application number
EP 79 10 2136

## DOCUMENTS CONSIDERED TO BE RELEVANT

| Category | Citation of document with indication, where appropriate, of relevant passages | Relevant to claim |
|---|---|---|
| X | GB - A - 1 493 006 (CIBA-GEIGY)<br>* Claims 1,15,17,38-39, 75-76 *<br>-- | 1,3-8 |
| DX | GB - A - 1 465 946 (CIBA GEIGY)<br>* Claims 23-29,44; page 4, lines 4-31 *<br>-- | 1,3-6, 8 |
| D | US - A - 4 053 605 (J. BALDWIN)<br>-- | 1,2,8, 9 |
| D | BE - A - 858 867 (CIBA-GEIGY)<br>* Claims; examples I,VIII *<br>---- | 1,8,9 |

**CLASSIFICATION OF THE APPLICATION (Int. Cl. [3])**

C 07 D 213/85
405/12
A 61 K 31/44//
C 07 C 91/18

**TECHNICAL FIELDS SEARCHED (Int.Cl. [3])**

C 07 D 213/85
405/12
A 61 K 31/44

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 28-09-1979 | NUYTS |